(19) **Europäisches Patentamt / European Patent Office / Office européen des brevets**

(11) **EP 4 706 527 A1**

(12) **EUROPEAN PATENT APPLICATION**

(43) Date of publication:
**11.03.2026 Bulletin 2026/11**

(21) Application number: **24199102.5**

(22) Date of filing: **08.09.2024**

(51) International Patent Classification (IPC):
**A61B 5/145** (2006.01)  **A61B 5/1468** (2006.01)
**A61B 5/1495** (2006.01)  **A61B 5/00** (2006.01)

(52) Cooperative Patent Classification (CPC):
**A61B 5/14532; A61B 5/14546; A61B 5/1468;
A61B 5/1495; A61B 5/4845;** A61B 2503/04;
A61B 2562/06

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Universität Zürich**
**8006 Zürich (CH)**

(72) Inventors:
• **Di Costanzo Mata, Aldo**
**8006 Zürich (CH)**

• **Ding, Yun**
**8006 Zürich (CH)**
• **Ustinov, Oleksii**
**8006 Zürich (CH)**
• **Wolf, Martin**
**8006 Zürich (CH)**
• **Seitz, Peter**
**8006 Zürich (CH)**
• **DeMello, Andrew**
**8006 Zürich (CH)**

(74) Representative: **Schulz Junghans
Patentanwälte PartGmbB
Großbeerenstraße 71
10963 Berlin (DE)**

(54) **SENSOR HEAD FOR MEASURING THE CONCENTRATION OF AN ANALYTE IN THE BLOOD OR TISSUE OF AN ANIMAL OR A HUMAN, PARTICULARLY A PREMATURE INFANT, IN A SELF-CALIBRATING MANNER**

(57) The present invention relates to a sensor head (10) for measuring the concentration of an analyte in the blood or tissue of an animal or a human, which analyte passively diffused through the skin (2) of said animal or human (P), particularly a premature infant, comprising: a first membrane (30) having a first permeability with respect to the analyte, wherein the first membrane (30) comprises a first contact surface (30a) configured to contact the skin (2) to receive said analyte, a second membrane (31) having a second permeability with respect to the analyte, wherein the second membrane (31) comprises a second contact surface (31a) configured to contact said skin (2) to receive said analyte, and wherein the first permeability differs from the second permeability, wherein the first contact surface (30a) is arranged besides the second contact surface (31a), wherein the first and the second contact surface (30a, 31a) are interleaved.

EP 4 706 527 A1

**Description**

The present invention relates to a sensor head for a sensor for measuring the concentration of an analyte in the blood or tissue of an animal or a human, particularly in the blood or tissue of a premature infant. Further aspects of the present invention relate to a sensor as well as to a corresponding method.

Particularly, WO2016008898A discloses a non-invasive self-calibrating analyte monitoring device designed for continuous use on humans and animals in blood or tissues. This monitoring device is able to measure all analytes that pass the skin barrier. In short, the self-calibrating nature of the device relies on two collectors that collect the analyte passing through the skin. The difference between the two collectors is the permeability of their membranes that is at the interface between skin and the collector.

In case the skin permeability to the analyte is the same for both collectors, then one has enough data to determine both the permeability of the skin and the analyte concentration.

However, measurements show that the assumption that the skin permeability is the same for the two collectors is not always true. Particularly, it has been found that for the majority of babies, the skin resistance was different between the two separate glucose collectors. Therefore, it is crucial to ensure an identical skin resistance value for both glucose collectors as the self-calibrating method only works accurately, if the skin resistance is essentially the same for both glucose collectors.

Based on the above, the problem underlying the present invention is to provide an improved sensor head, as well as a sensor and a method that alleviate the above-stated difficulty.

This problem is solved by a sensor head having the features of claim 1, by a sensor having the features of claim 12, and by a method having the features of claim 15.

Preferred embodiments of these aspects of the present invention are stated in the corresponding dependent claims.

According to claim 1, a sensor head for a sensor for measuring the concentration of an analyte in the blood or tissue of an animal or a human is disclosed, which analyte passively diffused through the skin of said animal or human, particularly a premature infant, wherein the sensor head comprises:

- a first membrane having a first permeability with respect to the analyte, wherein the first membrane comprises a first contact surface configured to contact said skin to receive said analyte,

- a second membrane having a second permeability with respect to the analyte, wherein the second membrane comprises a second contact surface configured to contact said skin to receive said analyte, and wherein the first permeability differs from the second permeability,

wherein the first contact surface is arranged besides the second contact surface, and wherein the first and the second contact surface are interleaved. Particularly, each of the first and second contact surface is continuous. Furthermore, particularly, due to the interleaved configuration, the first and the second membrane sample from essentially the same area of the skin of said human or animal. In other words, a bottom side of the sensor head that is configured to contact a skin area of the human or animal can be divided into a plurality of regular segments, wherein each segment comprises a portion of the first contact surface as well as a portion of the second contact surface.

In certain preferred embodiments, the sensor head may comprise a plurality of contact surfaces (and associated chambers, see below) having a number of contact surfaces (and particularly chambers) being larger than two, particularly 3 to 10 such contact surfaces (and associated chambers). Also, here, all contact surfaces are interleaved with one another to allow a distribution of each contact surface over the whole bottom side / skin contact area of the sensor head.

Particularly, due to said permeabilities being different, the mass flow rate of the analyte that diffuses through the first membrane when its first contact surface contacts the skin, differs from the mass flow rate of the analyte that diffuses through the second membrane when the second membrane contacts the skin. Notably, due to fact that the two contact surfaces are interleaved, they can efficiently share a skin area of the patient such that each contact surface/membrane sees essentially the same skin resistance to the flow of the analyte. This greatly improves the accuracy of the sensor.

In this way, precise analyte concentrations for the first and the second permeability can be acquired, particularly at the same time, which allows to calculate the concentration of the analyte in the blood (or skin) of the human or animal patient (without further calibration). Furthermore, particularly, the sensor according to the invention is a self-calibrating sensor as the two analyte concentrations determined for the two membranes suffice to determine the desired analyte concentration in the blood or tissue of the patient. Particularly, the present invention can be used with all analytes that can diffuse through the skin, preferably glucose, but also caffeine, urea, cortisone, hydrocortisone, lactate, opioids, cocaine, ammonia, creatinine, bilirubin.

Particularly, the first and the second membrane can be arranged adjacent one another, but do not overlap in a direction normal to the respective contact surface. Furthermore, in a preferred embodiment, the first and the second membrane are semi-permeable membranes.

According to a preferred embodiment of the present invention, a portion of the first contact surface is arranged between two portions of the second contact surface (or vice versa) that are opposite one another in a first direction, wherein particularly the first direction extends parallel to the first and second contact surface.

Further, in a preferred embodiment, the first and the second contact surface interlock.

According to yet another preferred embodiment, the first contact surface is u-shaped. Likewise, in a preferred embodiment, the second contact surface is u-shaped, too, particularly allowing the contact surfaces of the membranes to mutually interlock.

According to a further embodiment, wherein the first contact surface is comb-shaped. Furthermore, in a preferred embodiment, the second contact surface is and/or wherein the second contact surface comb-shaped.

Further, in a preferred embodiment, the first contact surface comprises a spiral shape and/or the second contact surface comprises a spiral shape.

According to a further preferred embodiment, the first contact surface comprises piecewise straight edges and/or the second contact surface comprise piecewise straight edges.

According to yet another preferred embodiment, the first contact surface comprises a curved edge and/or wherein the second contact surface comprises a curved edge.

Furthermore, in a preferred embodiment, the first contact surface comprises two elongated segments that are integrally connected at an end of the first contact surface and extend along one another separated by a gap. Likewise, in the same fashion, the second contact surface can comprise two elongated segments that are integrally connected at an end of the second contact surface and extend along one another separated by a gap.

According to yet another preferred embodiment of the present invention, the sensor head comprises a first chamber adjacent to the first membrane for receiving said analyte and a second chamber adjacent to said further membrane for receiving said analyte, wherein particularly the first chamber comprises a first inlet for feeding a perfusion medium into the first chamber, and a first outlet for discharging said perfusion medium out of the first, particularly so as to transport said analyte diffusing through the first membrane out of the first diffusion chamber, and wherein particularly the second chamber comprises a second inlet for feeding a perfusion medium into the second chamber, and a second outlet for discharging said perfusion medium out of the second chamber, particularly so as to transport said analyte diffusing through said second membrane out of the second chamber.

Particularly, the first and the second chamber are interleaved as well, particularly corresponding to the configuration of the first and second contact surfaces of the membranes.

Particularly, in a preferred embodiment, the first chamber comprises a first opening towards the first membrane, wherein the first membrane (and its first contact surface) covers the first opening of the first chamber. Similarly, in a preferred embodiment, the second chamber comprises a second opening towards the second membrane, wherein the second membrane (and its second contact surface) covers the second opening of the second chamber. In a preferred embodiment, the first opening of the first chamber and the second opening of the second chamber are interleaved, wherein the first opening follows the course of the first contact surface and the second opening follows the course of the second contact surface. Correspondingly, in certain embodiments, the first and the second opening can comprise the features described above with respect to the interleaved configuration of the first and the second contact surface.

Furthermore, in a preferred embodiment, the first and the second membrane are arranged on a bottom side of the sensor head, via which bottom side the sensor head is configured to be applied to a patient's skin, so that the contact surfaces of the membrane tightly contact the skin. Particularly, the sensor head, particularly its bottom side can comprise a certain flexibility to conform to a curvature of the skin of the patient.

Particularly, in a preferred embodiment, the sensor head comprises an (e.g. monolithic) main body accommodating the first and the second chamber, wherein the first and the second membrane are connected (particularly bonded) to the main body. The first chamber can be formed by a first recess arranged in the main body, wherein the first recess can be covered by the first membrane to enclose the first chamber. Similarly, the second chamber can be formed by a second recess arranged in the main body, wherein the second recess can be covered by the second membrane to enclose the second chamber.

In a preferred alternative embodiment of the present invention, the sensor head comprises a first body accommodating the first chamber and a separate second body accommodating the second chamber, wherein the first and the second body are connected to one another and are interleaved. Also, here, the first chamber can be formed by a first recess arranged in the first body, wherein the first recess can be covered by the first membrane to enclose the first chamber. Similarly, the second chamber can be formed by a second recess arranged in the second body, wherein the second recess can be covered by the second membrane to enclose the second chamber.

According to a further preferred embodiment, the sensor comprises an analyzing device for measuring the concentration of said analyte in the blood or tissue of said animal or said human, wherein said analyzing device is designed to measure a first concentration of the analyte diffused through the first membrane, and wherein said analyzing device is designed to measure a second concentration of the analyte diffused through the second membrane, and wherein particularly the analyzing means is designed to measure the concentration of the analyte in the blood or tissue using said first and second concentration. Particularly, the sensor head can be used with different analyzing devices, depending on the analyte and the preferred method of analyte detection.

Particularly, based on experiments conducted on various sensors, the sensor's underlying mathematical model has been enhanced. Initially, the system conformed to a self-calibrating approximation as outlined in equation (5) of WO2016008898A1. However, it has been found out that this approximation only holds for steady-state behavior. Given the possible instability of glucose levels of patients, particularly due to their prematurity, a detailed analysis of the sensor's response during transitional states has been conducted. Particularly, as a consequence, the model has been adapted to a first-order system behavior, wherein the first-order system behavior applies per chamber/membrane, i.e., the sensor head comprises two (or more) first-order systems that define the self-calibrating formula described below, wherein, the subscript "1" of the respective quantity refers to the first membrane, and "2" to the second membrane,

$$V_{BGC} = \frac{Q}{A}(R_1 - R_2)\frac{V_1(t)V_2(t)}{V_2(t)\left(1 - e^{-(t-t_1)\left(\frac{Q}{C_1 A}\right)}\right) - V_1(t)\left(1 - e^{-(t-t_2)\left(\frac{Q}{C_2 A}\right)}\right)}$$

wherein $V_{BGC}$ is the desired concentration of the analyte (e.g. glucose) in the blood or tissue of the patient to be calculated by the analyzing device. $V_1(t)$ is the first concentration of the analyte (measured by the analyzing device) that diffused through the first membrane and $V_2(t)$ is the second concentration of the analyte (measured by the analyzing device) that diffused through the second membrane. Particularly, $V_{BGC}$, $V_1(t)$, and $V_2(t)$ are in molar concentration units, in [mmol/L].

Furthermore, $R_1$ and $R_2$ are the diffusion resistances of the first and the second membrane, particularly in [s/cm], wherein $R_1$ is assumed to be larger than $R_2$.

Further, $C_1$ and $C_2$ represent the capacitive effect on the sensor head (particularly in [cm]), particularly at the respective membrane site since it shows "charging/emptying" behavior similar to an electric capacitance, potentially reflecting the effective membrane thickness.

Furthermore, $t_1$ and $t_2$ account for the time delays from when the sensor head first contacts the analyte source to when the analyte (contained in the dialysate) is available for detection, either inside the respective chambers (e.g. for internal or embedded quantification systems like functionalized electrodes) or at the outlet of the respective chamber (e.g. for external quantification systems like the measuring device described herein, e.g. microfluorimeter, particularly microfluidic chip).

Further, Q represents the flow applied across the respective chamber of the sensor head, in [cm³/s], and A represents the active collection area of the sensor head (particularly in [cm²]), i.e., the area actively collecting the analyte (e.g. glucose), which is the opening of the respective chamber covered by the respective membrane/contact surface (see also above).

Preferably, the above equation is used in the sensor's analysis device in the following form:

$$V_{BGC} = \frac{Q}{A}(R_1 - R_2)\frac{V_1(t)V_2(t)}{V_2(t)\left(1 - \frac{V'_1(t)}{V'_1(t) + \frac{Q}{C_1 A}V_1(t)}\right) - V_1(t)\left(1 - \frac{V'_2(t)}{V'_2(t) + \frac{Q}{C_2 A}V_2(t)}\right)}$$

wherein $V'_1(t)$, and $V'_2(t)$ represent the change of molar concentration over time of the first concentration of the analyte and the second concentration of the analyte, particularly in [(mmol/L)/s].

Furthermore, in a preferred embodiment, the analyzing device is configured to be connected to said first and second outlet of the chambers of the sensor, so that the respective perfusion medium together with the respective analyte can be transported to the analyzing device.

According to a preferred embodiment of the sensor, the analyzing device comprises a at least a first measuring device such as a first microfluidic chip, configured to measure the first concentration of the analyte using e.g. a fluorescence measurement, which first measuring device (e.g. first microfluidic chip) can be integrated into the sensor, particularly into the sensor head. Furthermore, according to a preferred embodiment, the analyzing device comprises a second measuring device (e.g. microfluidic chip) configured to measure the second concentration of the analyte using e.g. a fluorescence measurement (e.g. as described in de Courten, D., Baumann, L., Scherer, L. J., & Wolf, M. (2012). Opto-Fluidic Chip for Continuous Inline Monitoring of Glucose with Kinetic Enzymatic Fluorescence Detection. Procedia Engineering, 47, 1203-1206), which second measurement device (e.g. second microfluidic chip) is preferably integrated into the sensor, particularly into the sensor head. Thus, the first and the second analyte concentration can be measured in parallel. However, the device may also comprise an analyzing device (having e.g. a single measuring device, particularly a single microfluidic chip) configured to determine the first and the second analyte concentration sequentially. It is to be noted that the sensor head according to the present invention can be used with a sensor that can use any suitable method/technique to measure the concentration of an analyte that has diffused through the respective membrane into the respective chamber of the sensor head. Such methods/techniques are not limited to the above- mentioned fluorescence measurements and may be chosen depending on the respective analyte (particularly glucose) or other preferences.

According to yet another preferred embodiment, the analyzing device comprises a computer on which a suitable computer program is executed comprising program code that is adapted for determining the concentration of the analyte in the blood or tissue of the human or animal using the two measured concentrations, e.g., based on one of said above-stated formulas (see also above).

Further, according to a preferred embodiment of the sensor head / sensor, the first membrane and/or the second membrane comprises one of: poly(hydroxyethyl methacrylate) (HEMA), an hydrogel-forming acrylate, methacrylic acid (MMA), aminoethyl methacrylate (AEMA), hydroxyethyl acrylate (HEA), methacryloxypropyl-terminated poly(dimethylsiloxane) (PDMS), an acrylate-terminated PDMS.

Particularly, a polymer ratio of the respective substance is tuned to achieve the desired analyte permeability of the respective membrane.

Further, according to a preferred embodiment the respective first or second membrane comprises a hydroxyacrylate:PDMS ratio (in particular HEMA:PDMS ratio) in the range from 70:30 to 30:70 w/w, particularly to create a first and a second membranes with different (high and low) analyte permeability.

According to yet another preferred embodiment of the sensor head / sensor, the first and/or the second membrane comprises a thickness in the range from 30 to 120 $\mu$m, particularly depending on the required mechanical properties and permeability of the respective membrane.

According to a further preferred embodiment of the sensor head / sensor, other biocompatible semi-permeable materials can be used for the first and/or second membrane, such as one of: polycarbonate (PC), cellulose acetate (CA), polyethersulfone (PES), polyethylene terephthalate (PET). Particularly, in an embodiment, the first and/or second membrane can comprise a pore size in the range from 0.01 to 1.0 $\mu$m to regulate analyte passage.

In certain embodiments of the sensor head / sensor, alternative materials for the membranes like one of: polytetrafluoroethylene (PTFE), polyvinylidene fluoride (PVDF), polyacrylonitrile (PAN), polyamide (PA) may also be considered.

According to a further aspect of the present invention, a method for measuring the concentration of an analyte in the blood or tissue of an animal or a human, particularly a premature infant, is disclosed wherein the method comprises the steps of:

- letting the analyte diffuse through a first membrane comprising a first permeability with respect to said analyte, wherein the first membrane comprises a first contact surface for contacting a skin of the animal or human, and

- letting said analyte diffuse through a second membrane comprising a second permeability with respect to said analyte, wherein the second membrane comprises a second contact surface for contacting said skin, wherein the first permeability differs from the second permeability, and wherein the first and the second contact surface are interleaved, and

- measuring a first concentration of the analyte diffused through the first contact surface of the first membrane, and measuring a second concentration of the analyte diffused through the second contact surface of the second membrane, and determining the concentration of said analyte in the blood or tissue using said first and second concentration.

Preferably, the method according to the present invention uses a sensor head and/or sensor according to the present invention. Thus, particularly, the method according to the present invention may be further characterized based on the embodiments and features of the sensor head and/or sensor according to the present invention described and claimed herein.

Preferably, the analyte (that e.g. passively diffused through the skin of the human or animal patient) is collected in the first chamber of the sensor head via the first membrane (e.g. in a perfusion medium) by contacting the skin of the patient with the first contact surface of the first membrane so that said analyte diffuses through the first membrane. Alternatively, the analyte may diffuse from the first chamber (e.g. out of the perfusion medium) through the first membrane towards the skin (here also a specific first concentration of the analyte is established in the perfusion medium which in this case contains the analyte right from the start, see above). The first permeability may be higher than the second one (or vice versa).

Preferably, the perfusion medium showing the first concentration of the analyte is transported to the analyzing device, particularly by pumping the perfusion medium through the first chamber which takes along the analyte to the analyzing device.

Preferably, the first concentration of the analyte in the perfusion medium is measured and particularly stored by the analyzing device.

Furthermore, preferably, the analyte (that e.g. passively diffused through the skin of the patient) is also collected in the second chamber of the sensor head via the second membrane (e.g. in a perfusion medium) by contacting the skin with the second contact surface of the second membrane so that the analyte diffuses through the second membrane.

Collecting the analyte in the first and second chamber can in principle be done simultaneously, i.e. in parallel.

Preferably, the perfusion medium showing the second concentration of the analyte (being lower or higher than the first concentration) is transported to the analyzing device, particularly by pumping the perfusion medium through the second chamber which takes along the analyte to the analyzing device.

Preferably, the second concentration of the analyte in the fluid perfusion medium is measured. In principle, the two concentrations of the analyte may be measured simultaneously, i.e. in parallel. In this case, the analyzing device can comprise the components needed for each chamber (e.g. two measuring devices / microfluidic chips).

Preferably, the concentration of the analyte in the blood (e.g. glucose) or skin is then determined by the analyzing device using the two measured concentrations, e.g. as described above by using one of the above-stated formulas.

Advantageously, the method according to the invention is non-invasive for very permeable skin, as the one of preterm infants, or for measuring analytes to which the skin is very permeable (i.e. all molecules that go through the untreated skin in measurable amount, such as e.g. glucose for the preterm neonate). In this case, no invasive methods (e.g. microneedles) at all are necessary for measuring the blood analyte concentration.

However, the method according to the invention may also be used with lesser permeable skin. Here, prior to conducting the method according to the invention, the skin of the patient may be prepared with minimally invasive methods (e.g. microneedles puncturing the skin, skin abrasion, thinning of the stratum corneum by microdrilling with a laser array or other chemical, electromagnetic, thermal and mechanical methods) in order to make the skin sufficiently permeable for the desired analytes.

Further, the skin may be prepared in order to increase its permeability prior to measuring the desired analyte according to the invention by means of sonophoresis, electrophoresis, or by removing the superficial skin layers mechanically or chemically.

Further features and advantages of the invention shall be described by means of a detailed description of embodiments with reference to the Figures, wherein

Fig. 1    shows a schematical view (not to scale) of an embodiment of the sensor head and particularly sensor according to the invention;

Fig. 2    shows a schematical cross section (not to scale) of a sensor head according to the present invention;

Fig. 3A-3I    shows a schematic view of interleaved contact surfaces of the membranes of embodiments of the sensor head according to the present invention;

Fig. 4A    shows a perspective view of a first body of a sensor head according to the present invention;

Fig. 4B    shows a perspective view onto the contact surfaces of the embodiment shown in Fig. 4A;

Fig. 5    shows a schematic illustration of an analyzing device of an embodiment of a sensor according to the present invention, and

Fig. 6    shows a top view onto a measuring device (here microfluidic chip) that can be used in conjunction with a sensor head / sensor according to the present invention.

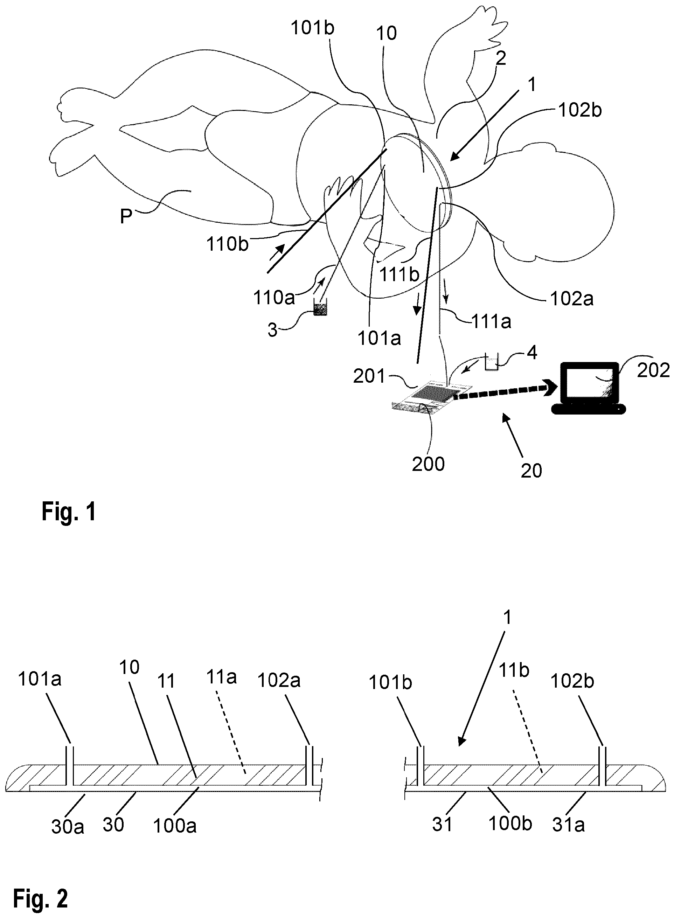

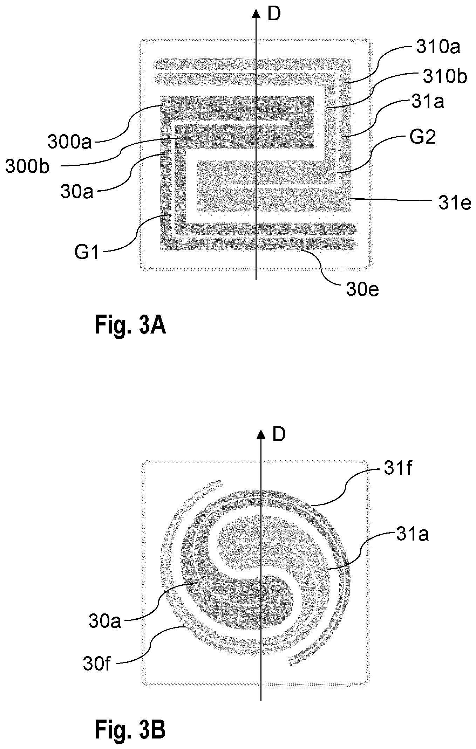

Fig. 1 shows in conjunction with Fig. 2 a schematic illustration of an embodiment of a sensor head 10 according to the present invention. The sensor head 10 facilitates measuring the concentration of an analyte in the blood or tissue of an animal or a human P, particularly a premature infant, and comprises a first membrane 30 having a first permeability with respect to the analyte, wherein the first membrane 30 comprises a first contact surface 30a configured to contact a skin 2 of said patient P to receive said analyte. Further, the sensor head 10 comprises a second membrane 31 having a second permeability with respect to the analyte, wherein the second membrane 31 comprises a second contact surface 31a configured to contact said skin 2 to receive said analyte, wherein the first permeability differs from the second permeability, and wherein the first contact surface 30a is arranged besides the second contact surface 31a (cf. Fig. 2), wherein the first and the second contact surface 30a, 31a are interleaved. Different embodiments of this interleaved structure are exemplary shown in Figs. 3A to 4B and will be described in more detail below. Particularly, the advantage is that the interleaved design of the contact surfaces 30a. 31a significantly enhances the accuracy of analyte measurement by ensuring that both membranes 30, 31 sample from essentially the same area of skin. This configuration minimizes the impact of local variations in skin permeability for the analyte, allowing for more reliable self-calibration and thus more accurate determination of analyte concentrations in the blood or tissue.

Particularly, the sensor head 10 further comprises a first and a second chamber 100a, 100b (also denoted as microdialysis chamber) each being delimited by the associated first or second membrane 30, 31 as e.g. indicated in Fig. 2 that serve for contacting the skin 2 of the patient P via the respective contact surface 30a, 31a. The respective membrane 30, 31 is arranged on a bottom side of the sensor head 10. The respective chamber 100a, 100b comprises an inlet 101a, 101b as well as an outlet 102a, 102b for feeding a perfusion medium 3 into the respective chamber 100a, 100b and for discharging it out of the respective chamber 100a, 100b, wherein the respective outlet 102a, 102b is configured to be connected to an analyzing device 20 for measuring the concentration of an analyte (e.g. glucose) in a perfusion medium 3, which analyte diffused through the respective membrane 30, 31. In this way the sensor head 10 can be complemented to form a sensor 1 for measuring the concentration of the analyte in the blood or tissue of a patient P. As indicated in Fig. 1, the perfusion medium 3 can be analysed in the analyzing device 20 sequentially, i.e., at first the perfusion medium 3 coming from the first chamber 100a is analysed to determine the first concentration of the analyte and then the perfusion medium 3 coming from the second chamber 100b may be analysed to determine the second concentration of the analyte. Alternatively, the necessary components of the analyzing device 20 can be provided for each chamber 100a, 100b to carry out the determination of the first and the second analyte concentration in parallel.

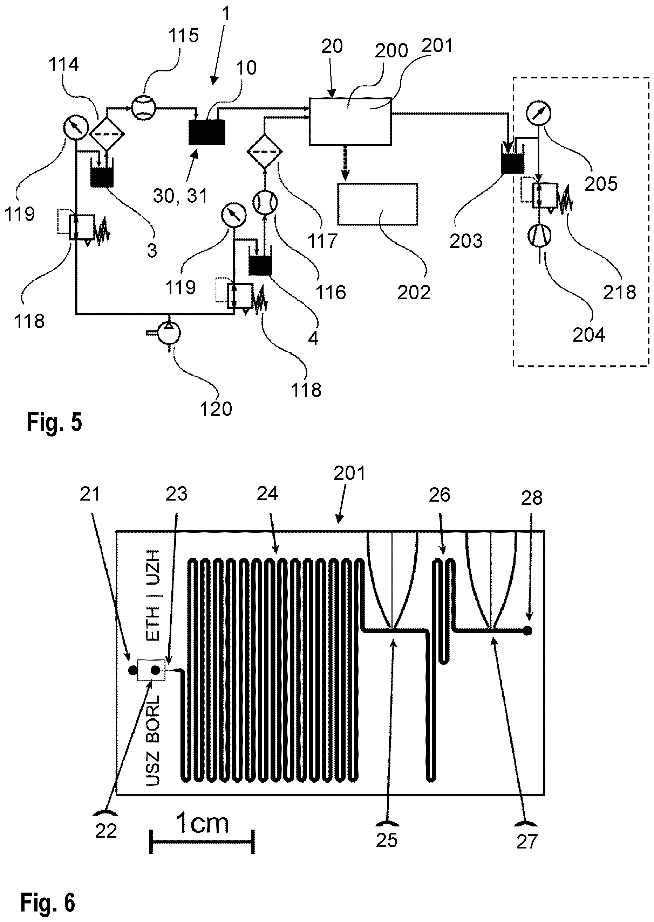

Further, reagents 4 can be provided for the ex-vivo measurement of the concentration of the analyte in said mixture with the perfusion medium 3 that flows to the analyzing device 20. For measuring said concentrations, the analyzing means 20 preferably comprises at least one measuring device, e.g. a microfluorimeter (optional if a different concentration measurement is chosen) 200. or two measuring devices/microfluorimeters for carrying out the analysis in parallel (see above), wherein the respective microfluorimeter preferably comprises a microfluidic chip 201 (cf. Fig. 6) that determines the analyte concentration(s) in the perfusion medium, as well as a computer 202 or a dedicated embedded computer for computing the analyte (e.g. glucose) concentration in the blood/tissue of the patient B from the two analyte concentrations in the perfusion media, and for displaying it.

Fig. 2 shows a schematical cross section of the sensor head 10 of the device 1 according to the invention. The sensor head 10 can comprise a main body 11 formed e.g. out of PDMS for accommodating the chambers 100a, 100b. Alternatively, the sensor head 10 may comprise a separate body (e.g. PDMS) for each chamber 100a, 100b which are connected to one another, particularly in an interleaved fashion. When the probe head 10 contacts the skin 2 of the patient P via the respective contact surface 30a, 31a of the membranes 30, 31, said analyte (e.g. glucose) of the ex-vivo body fluid of the patient P can diffuse through the respective membrane 30, 31, which delimits the corresponding chamber 100a, 100b, into the respective chamber 100a, 100b.

As shown in Figs. 1 and 2 the sensor 1 or sensor head 10 can further comprise conduits 110a, 100b to establish flow connections with the first and the second inlet 101a, 101b as well as conduits 11 1a, 111b to establish flow connections to the first and second outlet 102a, 101b. Via these conduits, the respective perfusion medium 3 can be transported to the analyzing device 20 / measuring devices (e.g. microfluidic chip(s) 201).

For determining the actual concentration of the analyte in the blood of the patient P, the ex-vivo analytes passively diffusing through the skin 2 and the membranes 30, 31 are analyzed. For this, these analytes are carried away from the sensor head 10 to the analyzing device 20 by means of the perfusion media 3. Appropriate analysis allows the measurement of analyte concentration in the respective perfusion medium 3 coming from the first or second chamber 100a, 100b from which the blood analyte level can be calculated.

For glucose analysis, the glucose containing perfusion medium/fluid 3 can be mixed with an enzymatic solution for the stoichiometric conversion of glucose by the analyzing device 20. This final solution can then be spectroscopically analyzed by a fluorimeter of the analyzing device 20. This is transferred into the microfluidic chip 201 connected to the outlet 102 of the probe head 10 as shown in Figs. 1 and 5. Figure 5 shows a block diagram of an embodiment of the sensor 1 according to the present invention. The perfusion medium 3 is pumped via a particle filter 114 and a liquid flow meter 115 through the respective inlet 101a, 101b into the respective diffusion chamber 100a, 100b of the sensor head 10 and transports the analyte to the analyzing device 20, namely to the microfluidic chip 201 of the microfluorimeter 200 that is designed to measure the glucose concentration in the respective perfusion medium 3. Since a known flow of perfusion medium 3 is used, the concentration of the analyte in the respective fluid 3 can be determined by the analyzing device 20. Further, the reagents or reagent fluid 4 (preferably a Hexokinase/Glucose-6-phosphate dehydrogenase/ATP/buffer/Mg2+ solution in water) for the ex-vivo measurement of the concentration that flows to the microfluorimeter 200 (both optional if a different concentration measurement is chosen; the analyzing means should just allow to monitor preferably continuously (or almost) the concentration within the required range with sufficient precision) are pumped via a flow meter 116 and a particle filter 117 to the microfluorimeter 200 of the analyzing device 20.

The microfluidic chip 201 that determines the respective glucose concentration is connected to a computer 202 or a dedicated embedded computer for computing the glucose concentration from the two concentrations for the two membranes as measured by the microfluorimeter 200. Again, the perfusion media 3 from the chambers 101a, 101b can be measured one after the other or the above-described components are provided for each chamber 101a, 101b separately so that the analysis can be done in parallel. The computer 202 can be further designed to display the computed concentration of the analyte in the blood of the patient P. Analyzed dialysate is discharged into a waste container 203.

Further, particularly, the air compressor 120 or compressed air line, the precision air pressure regulators 118 (e.g. with feedback and vent), as well as the air pressure measurement devices 119 allow a pressure driven flow of the perfusion medium 3 through the sensor head 10/ chamber 100a, 100b into the microfluorimeter 200 and of the reagent fluid 4 into said microfluorimeter 200 with a very low pressure variation. The pressure from the air compressor/compressed air line 120 is brought to two the two precision pressure regulators 118 so that the two reservoirs 3, 4 for the reagent fluid and the perfusion medium (or perfusate) are pressurized. Controlling now the pressure difference of each reservoir 3, 4 in comparison with the pressure of the analyzing device outlet (at the atmospheric pressure) the pressure difference will drive laminar flows into the sensor head 10, tubing, and the microfluidic chip 201.

Furthermore, as indicated with dashed lines, the sensor 1 can comprise an additional vacuum pump 204 connected via a pressure regulator 218 to the waste container 203 for receiving the analyzed dialysate. The pressure generated by the pump 204 can be measured by a pressure measurement device 205. The reagent fluid container could also be connected to this additional vacuum pump 204 via a pressure regulator instead of the air compressor/compressed air line 120.

Then, with measuring the two analyte concentrations for two different membranes 30, 31 one gets the blood glucose concentration $V_{BGC}$ from the above-stated formula.

Furthermore, the principle of the microfluidic chip 201 for concentration measurement by means of with fluorescence measurement is shown in Fig. 6. The chip 201 is preferably made out of polydimethylsiloxane (PDMS) that is molded onto a SU-8 on silicon mold fabricated by a non-standard photolithographic process described in detail in [1]. The chip 201 may also be made out of polycarbonate (PC), e.g., by means of injection molding. The chip 201 uses continuous flow rates. It first passively mixes the diluted analytes in the dialysate (e.g. in the respective mix with the perfusion medium 3) with reagent fluid 4 for the standard hexokinase glucose assay. The mixing duration with passive diffusion is negligible compared with the reaction lag time. Thus, the enzymatic reaction is not diffusion limited and follows a pseudo-first-order reaction kinetic. There is a delay channel (60 s) 24 for the lag time of the coupled enzyme reaction. The fabricated channel height is controlled and uniform so that the delays in the channels are not altered. With a channel uniformity of $\pm$ 2.5% on thick structures this is a non-standard fabrication process with multilayers UV patterned SU-8 photoresist using only the central part of the silicon wafer for the mold. The flow inside the channels is kept laminar so that little transverse mixing occurs, i.e., so that the flow is uniform over the channel length.

In detail, as shown in Fig. 6, the microfluidic chip 201 for monitoring the glucose concentration in the dialysate comprises a reagent inlet 21 for the reagent fluid 4, a dialysate inlet 22 used for said dialysates, a micromixer 23 for mixing dialysate and reagent fluid 4, said delay channel 24, a first fluorescence chamber 25, a second delay channel 26, a second fluorescence chamber 27, as well as a waste outlet 28 for discharging the measured dialysate.

After the first delay channel 24, the pseudo-first order reaction is monitored by the fixed-point method using the two fluorescence chambers 25, 27 separated by the 7 s second delay channel 26. The difference between the two fluorescence signals of the enzymatic reaction product is proportional to the glucose concentration.

In the above, the glucose measurement was described as one possible application of the device 1 according to the invention, because it has a tremendous clinical value. However, the same principle can be applied to any analyte that diffuses through the skin and thus the device 1 enables a broad new approach of non-invasive diagnostics.

The two contact surfaces 30a, 31a of the membranes 30, 31 can be interleaved to increase the accuracy of the sensor 1 in various different manners.

Particularly, as indicated in Fig. 3A to Fig. 4B due to being interleaved, there is at least a portion of the first contact surface 30a arranged between two portions of the second contact surface 31a that are arranged opposite one another in a first direction D that particularly extends parallel to the first and second contact surface 30a, 31a.

Such an interleaved configuration facilitates that the chambers 100a, 100b and their membranes can share the skin 2 of the patient P in a way that variations in the skin's resistance to the analyte can be averaged out as due to the interleaved configuration it becomes more likely that a local maximum or minimum of the resistance is seen by portions of both contact surfaces 30a, 31a.

Particularly, the contact surfaces 30a, 31a can mutually interlock as shown in Fig. 3A and may each comprise a u-shaped configuration as indicated in Fig. 3A.

Further, the contact surfaces 30a, 31a may each comprise a spiral shape as shown in Fig. 3B.

Furthermore, as particularly indicated in Figs. 3A and 3B, the first contact surface 30a can comprises two elongated segments 300a, 300b that are integrally connected at an end of the first contact surface 30a and extend along one another separated by a gap G1. Similarly, the second contact surface 31 can comprise two elongated segments 310a, 310b that are integrally connected at an end of the second contact surface 31a and extend along one another separated by a gap G2.

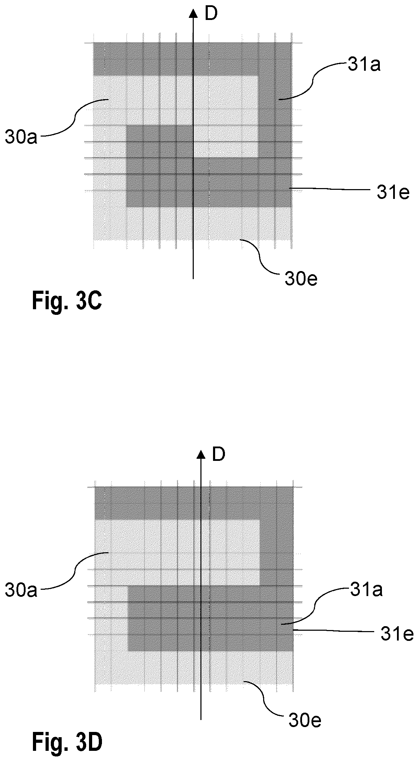

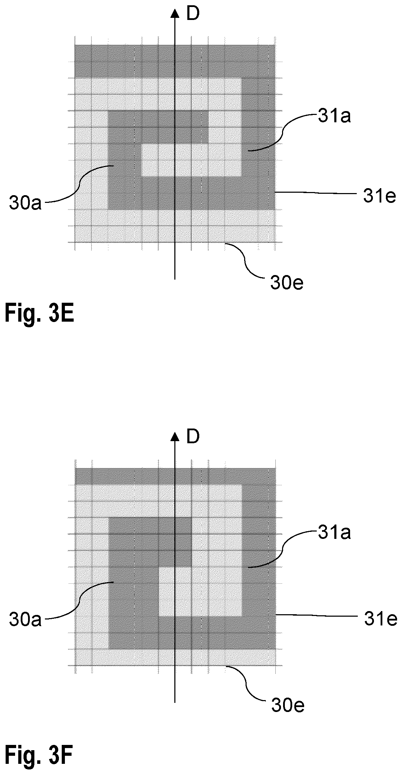

Figs. 3C to 3F show further u-shaped interlocking contact surfaces 30a, 31a, particularly spiral-shaped contact surfaces 30a, 31a (e.g. Figs. 3C, 3E, 3F).

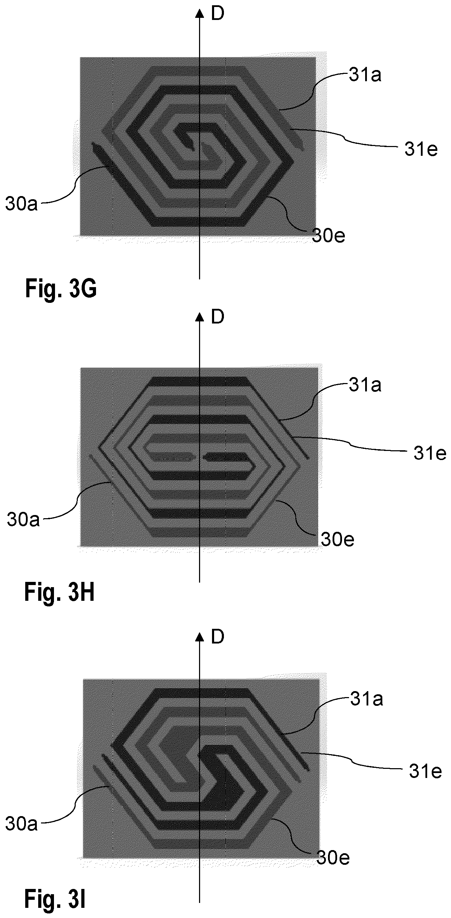

Further, spiral-shaped configurations are shown in Figs. 3G to 3I.

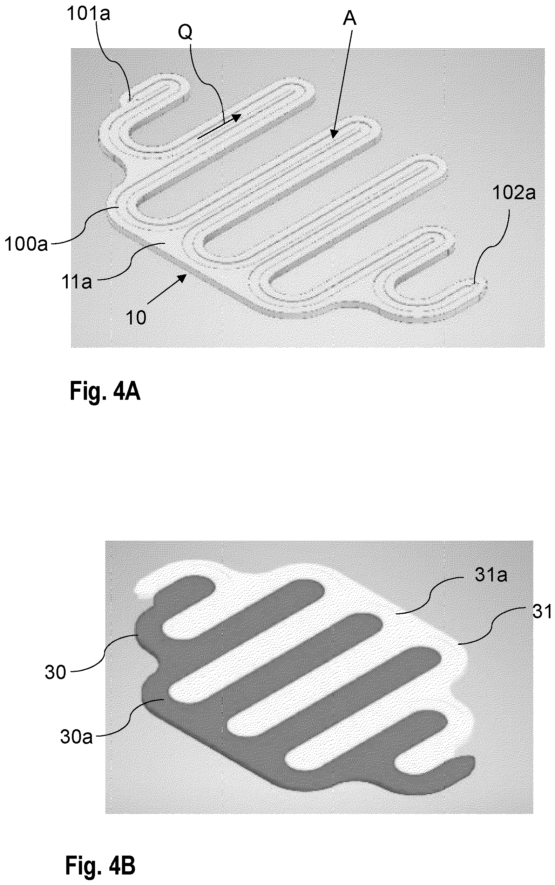

Furthermore, the contact surfaces 30a, 31a can comprise curved edges 30f, 31f as e.g. shown in Figs. 3B and 4B, but may also comprise piecewise straight edges 30e, 31e as e.g. shown in Fig. 3A, 3C, 3D, 3E, 3F, 3G, 3H, 3I.

Furthermore, Fig. 4A shows a first body 11a of a sensor head 10 for accommodating the first chamber 100a, which body 11a is configured to interleave with a second body (not shown) to form the sensor head 10.

The contact surfaces 30a, 31a of such a combination of interleaved bodies of the sensor head 10 can comprise the interlocking comb-shapes shown in Fig. 4B.

Furthermore, Fig. 4A indicates the first chamber 100a having an e.g. meandering shape and being formed by a recess arranged in the first body 11a. The first chamber/recess 100a comprises a first opening that is covered by the first membrane 30 as shown in Fig. 4B and comprises an area A via which the first chamber 100a can collect the analyte (e.g. glucose) that diffuses through the first contact surface 30a of the first membrane 30. The first chamber 100a (and its first opening) follow the course of the first contact surface 30a. The same applies to the second chamber and its second contact surface (not shown in Fig. 4B). Consequently, also the first and the second chamber 100a, 100b (and their first and second openings towards the respective membrane 30, 31) are preferably interleaved, e.g., as described herein with respect to the various embodiments.

Examples

In the following further examples of the present invention are described.

1. Main Body:

1.1 The primary material used for the collector's main body is medical-grade polydimethylsiloxane (PDMS, e.g., Dow Corning Sylgard 184 or equivalent), chosen for its transparency, flexibility, and biocompatibility.

1.2 Alternative materials include other skin-safe, flexible polymers such as medical-grade silicone elastomers (e.g., Dow Corning MED-4210), thermoplastic polyurethanes (e.g., Lubrizol Tecoflex), or parylene.

1.3 The main body material must provide a soft, conformable interface with the skin while maintaining adequate structural integrity.

1.4 Key characteristics: Shore A hardness of 40-60, transparency >60% in the 400-1100nm range, tensile strength >3 MPa.

2. Semi-Permeable Membranes:

2.1 The semi-permeable membranes are composed of a blend of poly(hydroxyethyl methacrylate) (HEMA) or other hydrogel-forming acrylates such as methacrylic acid (MMA), aminoethyl methacrylate (AEMA), hydroxyethyl acrylate (HEA), and methacryloxypropyl-terminated poly(dimethylsiloxane) (PDMS) and/or any other acrylate-terminated PDMS, with the polymer ratio tuned to achieve the desired analyte permeability.

2.2 HEMA/Acrylate:PDMS ratios ranging from 70:30 to 30:70 w/w are used to create membranes with high and low analyte permeability, respectively. Membrane thickness ranges from 30 to 120 $\mu$m, depending on the required mechanical properties and permeability.

2.3 Other biocompatible semi-permeable membrane materials, such as polycarbonate (PC), cellulose acetate (CA) polyethersulfone (PES), and polyethylene terephthalate (PET), may also be employed, with pore sizes ranging from 0.01 to 1.0 $\mu$m to regulate analyte passage. Additional materials like polytetrafluoroethylene (PTFE), polyvinylidene fluoride (PVDF), polyacrylonitrile (PAN), and polyamide (PA) may also be considered.

Fabrication Methods:

3. Microfluidic Channel Creation:

3.1 Soft lithography is the primary technique used to create microfluidic channels within the PDMS main body.

3.2 A master mold is created by patterning SU-8 photoresist on a silicon wafer using standard photolithography, with channel dimensions typically ranging from 50 to 500 $\mu$m in depth and width.

3.3 Example mold parameters: SU-8 2050 photoresist applied to a thickness of 50 $\mu$m, exposed to 365 nm UV light at 200 mJ/cm$^2$, developed in SU-8 developer for 10 min at room temperature.

3.4 PDMS (10:1 base to curing agent ratio) is poured over the mold, degassed, cured at 60-80°C for 1-2 hours, then peeled off.

3.5 Alternative methods like hot embossing, injection molding, or 3D printing (e.g. DLP with biocompatible resins), may be explored for scalability and cost-effectiveness.

4. Device Assembly:

4.1 The collector has two main parts: a high-permeability chamber and a low-permeability chamber, each bonded to the appropriate membrane.

4.2 The two halves are fabricated separately, then carefully aligned and assembled to form the complete interleaved device.

4.3 A final casting step may join the halves together seamlessly.

4.4 Alternatively, an integrated main body with both high and low permeability sections can be created in a single step, followed by selective membrane application.

5. Membrane-Body Bonding:

5.1 HEMA/PDMS or other membranes are bonded to the PDMS main body using plasma treatment, corona discharge, or chemical surface modification.

5.2 Oxygen plasma (50-100 W, 30-60 s) activates the PDMS and membrane surfaces for strong, irreversible bonding.

5.3 Chemical crosslinkers like 3-aminopropyltriethoxysilane (APTES) functionalize the membrane to form covalent bonds with PDMS.

5.4 APTES amino groups react with carboxyl or hydroxyl groups on polymer membranes to form stable amide bonds, while its triethoxysilane groups hydrolyze and condense to form strong siloxane bonds with PDMS hydroxyls, creating a durable membrane-PDMS interface.

5.5 For thermoplastic bodies, heat press-assisted molecular diffusion bonding at 80-100°C and 1-5 MPa for 10-30 min may be used.

Quality Control and Testing:
6. Visual Inspection:

6.1 Devices are visually inspected for surface smoothness, uniformity, and absence of defects or contamination.

6.2 Automated machine vision may verify channel dimensions and membrane integrity.

7. Mechanical Testing:

7.1 Destructive peel tests are performed on a sample of devices to quantify membrane-PDMS bond strength.

7.2 A minimum peel strength of 5 N/cm is required to ensure device integrity during use.

8. Fluidic Testing:

8.1 Devices are subjected to flow rates of 1-10 μL/min to check for leaks, blockages, pressure drops or delamination.

8.2 Bubble point tests assess the integrity of the semi-permeable membrane. The device is pressurized internally with air, while the exterior is wetted or immersed in liquid. The pressure at which bubbles first appear on membrane's external surface is recorded and compared to standards to ensure there are no defects.

9. Permeability Testing:

9.1 Differential analyte permeability of standalone membranes is first characterized using a Franz diffusion cell setup. An analyte solution at a physiologically relevant concentration (e.g., 1-20 mM for glucose) is placed in the donor compartment, and the receptor compartment is sampled over time to measure analyte flux. Permeability depends on the specific molecule, with glucose and caffeine showing notably different flux rates. The high-permeability membrane is designed to allow maximal analyte passage, while the low-permeability membrane should exhibit restricted permeability, similar to or lower than that of a biological barrier such as skin.

9.2 For assembled devices, overall analyte permeability is tested by exposing both sides to the same solution and measuring the flux across each membrane over time to ensure the differential permeability is maintained after integration.

Sterilization and Packaging:
10. Sterilization Process:

10.1 Assembled devices are rinsed with purified water to remove any manufacturing residues.
10.2 Devices are then subjected to UV sterilization (254 nm, 1-2 J/cm$^2$) to inactivate any microbial contaminants.
10.3 Prior to use, the device is wiped with a water-soluble skin-safe disinfectant (e.g. MeliseptolOsensitive). Alcohol-based disinfectants are avoided for acrylate membranes as they cause swelling and softening, disturbing the bond.

11. Packaging and Storage:

11.1 Sterilized devices are sealed in airtight, moisture-barrier packaging (e.g., aluminized polyester/polyethylene pouches) to maintain sterility during storage and transport.
11.2 Packaged devices are stored in a cool, dry place (15-30°C, 30-50% RH) and used within the designated shelf life (e.g., 12-24 months).
11.3 Once the packaging is opened, the device should be used immediately or resterilized if not used within a certain timeframe (e.g., 24 hours).

Scalability and Advanced Manufacturing Approaches:
12. Scale-Up Considerations:

12.1 For larger-scale production, injection molding or hot embossing with durable, reusable molds may be more suitable than soft lithography.
12.2 Automated assembly lines with precision alignment and bonding stations can help ensure consistency and efficiency in device fabrication.
12.3 Inline quality control systems, such as machine vision inspection and automated leak testing, can be integrated into the manufacturing process.

13. Challenges in Large-Scale Manufacturing:

13.1 The selective membrane bonding approach, where membranes with differential analyte permeability are bonded to specific regions of a single PDMS substrate, presents significant challenges in large-scale production. Achieving consistent and precise bonding across many units is complex and requires stringent process control.

13.2 Techniques such as masked plasma treatment, selective chemical functionalization, or precisely controlled multilayer casting must be carefully scaled up to ensure uniformity across batches.
Variability in these processes could lead to inconsistent membrane performance.

13.3 Continuous fabrication methods, such as roll-to-roll processing or multilayer coextrusion, could enable high-throughput production of the composite membrane-PDMS structures, but maintaining the integrity of the selective bonding process on a large scale is challenging.

13.4 While 3D printing technologies, such as multimaterial inkjet printing or stereolithography, offer flexibility in prototyping and small-batch customization, scaling these methods to mass production while maintaining precision and material properties poses significant hurdles.

Below, in Table 1, results are shown based on the above-stated approximation formula and the sensor head according to the present invention (last column on the right-hand side). The non-invasive, self-calibrating values could achieve up-to and within 1% from the reference values shown in the penultimate column on the right-hand side. This demonstrates the effectiveness of the approach according to the present invention

Table 1 - Glucose measurement results compared to invasive glucose measurement

| Patient ID | Gestational age (Weeks+days) | Postnatal age (days) | Gender | Invasive glucose measurement | Glucose measurement using sensor head |
|---|---|---|---|---|---|
| P1 | 32+0 | 2 | Male | 3.9 | - |

(continued)

| Patient ID | Gestational age (Weeks+days) | Postnatal age (days) | Gender | Invasive glucose measurement | Glucose measurement using sensor head |
|---|---|---|---|---|---|
| P2 | 32+3 | 2 | Male | 3.9 | 3.88 |
| P3 | 31+3 | 1 | Female | 4.8 | - |
| P4 | 32+3 | 0 | Female | 3.7 | - |
| P5 | 29+6 | 1 | Male | 7.8 | 7.72 |
| P6 | 29+6 | 1 | Male | 5.4 | 5.37 |
| P7 | 28+2 | 1 | Male | 12.4 | 12.41 |
| P8 | 28+2 | 1 | Female | 6.7 | 6.67 |
| P11 | 30+5 | 1 | Male | 4.4 | 4.42 |

Summarizing, the self-calibrating non-invasive sensor according to the present invention offers a groundbreaking approach to continuous monitoring of analytes in blood or tissues of humans and animals, particularly in preterm infants. By leveraging the unique permeability of preterm skin, the device enables accurate, real-time measurement of glucose, lactate, bilirubin and other biomarkers without invasive sampling.

The use of biocompatible materials, advanced fabrication techniques, and rigorous quality control aims to ensure the device's safety, reliability, and efficacy in neonatal settings. The innovative differential permeability design and specialized bonding methods facilitate the creation of a compact, user-friendly device that can be seamlessly integrated into existing neonatal care practices.

The glucose sensor according to the present invention has the potential to elevate the standard of care for preterm infants by minimizing the risks associated with invasive monitoring and managing glucose instability. Furthermore, this technology paves the way for non-invasive, continuous monitoring of other clinically relevant biomarkers, promising to enhance neonatal care and outcomes for this vulnerable patient population.

**Claims**

1. A sensor head (10) for a sensor (1) for measuring the concentration of an analyte in the blood or tissue of an animal or a human (P), comprising:

    - a first membrane (30) having a first permeability with respect to the analyte, wherein the first membrane (30) comprises a first contact surface (30a) configured to contact a skin (2) of said animal or human to receive said analyte,
    - a second membrane (31) having a second permeability with respect to the analyte, wherein the second membrane (31) comprises a second contact surface (31a) configured to contact said skin (2) to receive said analyte, and wherein the first permeability differs from the second permeability,

    wherein the first contact surface (30a) is arranged besides the second contact surface (31a), wherein the first and the second contact surface (30a, 31a) are interleaved.

2. The sensor head according to claim 1, wherein a portion of the first contact surface (30a) is arranged between two portions of the second contact surface (31a) which are opposite one another in a first direction (D), wherein particularly the first direction (D) runs parallel to the first and second contact surface (30a, 31a).

3. The sensor head according to one of the preceding claims, wherein the first and the second contact surface (30a, 31a) interlock.

4. The sensor head according to one of the preceding claims, wherein the first contact surface (30a) is u-shaped and/or wherein the second contact surface (31a) is u-shaped.

5. The sensor head according to one of the preceding claims, wherein the first contact surface (30a) is comb-shaped and/or wherein the second contact surface (31a) comb-shaped.

6. The sensor head according to one of the claims 1 to 3, wherein the first contact surface (30a) comprises a spiral shape and/or wherein the second contact surface (31a) comprises a spiral shape.

7. The sensor head according to one of the preceding claims, wherein the first contact surface (30a) comprises piecewise straight edges and/or wherein the second contact surface (31a) comprise piecewise straight edges (30e, 31e).

8. The sensor head according to one of the preceding claims, wherein the first contact surface (30a) comprises a curved edge (30f) and/or wherein the second contact surface (31a) comprises a curved edge (31f).

9. The sensor head according to one of the preceding claims, wherein the first contact surface (30a) comprises two elongated segments (300a, 300b) that are integrally connected at an end of the first contact surface and extend along one another separated by a gap (G1), and/or wherein the second contact surface (31) comprises two elongated segments (310a, 310b) that are integrally connected at an end of the second contact surface (31a) and extend along one another separated by a gap (G2).

10. The sensor head according to one the preceding claims, wherein the sensor head (10) comprises a first chamber (100a) adjacent to the first membrane (30) for receiving said analyte, and a second chamber (100b) adjacent to said second membrane (31) for receiving said analyte, wherein particularly the first chamber (30) comprises a first inlet (101a) for feeding a perfusion medium (3) into the first chamber (100a), and a first outlet (102a) for discharging said perfusion medium (3) out of the first chamber (30), and wherein particularly the second chamber (100b) comprises a second inlet (102a) for feeding a perfusion medium (3) into the second chamber (102a), and a second outlet (102b) for discharging said perfusion medium (3) out of the second chamber (100b).

11. The sensor head according to claim 10, wherein the sensor head (10) comprises a main body (11) accommodating the first and the second chamber (100a, 100b), wherein the first and the second membrane (30, 31) are connected to the main body (11), or wherein the sensor head (10) comprises a first body (11a) accommodating the first chamber (100a) and a separate second body (11b) accommodating the second chamber (100b), wherein the first and the second body (11a, 11b) are connected to one another.

12. A sensor (1) for measuring the concentration of an analyte in the blood or tissue of an animal or a human (P), wherein the sensor (1) comprises a sensor head (10) according to one of the preceding claims.

13. The sensor according to claim 12, **characterized in that** the sensor (1) comprises an analyzing device (20) for measuring the concentration of said analyte in the blood or tissue of said animal or said human, wherein said analyzing device (20) is designed to measure a first concentration $V_1(t)$ of the analyte diffused through the first membrane (30), and wherein said analyzing device (20) is designed to measure a second concentration $V_2(t)$ of the analyte diffused through the second membrane (31), and wherein particularly the analyzing means (20) is designed to determine the concentration $V_{BGC}$ of the analyte in the blood or tissue using said first concentration $V_1(t)$ and said second concentration $V_2(t)$, particularly based on the formula

$$V_{BGC} = \frac{Q}{A}(R_1 - R_2)\frac{V_1(t)V_2(t)}{V_2(t)\left(1-\frac{V\prime_1(t)}{V\prime_1(t)+\frac{Q}{C_1A}V_1(t)}\right)-V_1(t)\left(1-\frac{V\prime_2(t)}{V\prime_2(t)+\frac{Q}{C_2A}V_2(t)}\right)},$$

wherein $V\prime_1(t)$, and $V\prime_2(t)$ represent the change over time of said first and second concentration of the analyte, and wherein $R_1$, $R_2$, Q, A, $C_1$, $C_2$, $t_1$, and $t_2$ are real-valued numbers.

14. The sensor according to claim 13, **characterized in that** the analyzing device (20) is configured to be connected to said first and second outlet (102a, 102b), so that respective perfusion medium (3) together with the respective analyte can be transported to the analyzing device (20).

15. A method for measuring the concentration of an analyte in the blood or tissue of an animal or a human (P), particularly a premature infant, wherein the method comprises the steps of:

   - letting the analyte diffuse through a first membrane (30) comprising a first permeability with respect to said analyte, wherein the first membrane (30) comprises a first contact surface (30a) for contacting a skin (2) of the animal or human, and

- letting said analyte diffuse through a second membrane (31) comprising a second permeability with respect to said analyte, wherein the second membrane (31) comprises a second contact surface (31a) for contacting said skin (2), wherein the first permeability differs from the second permeability, and wherein the first and the second contact surface (30a, 31a) are interleaved, and

- measuring a first concentration of the analyte diffused through the first contact surface (30a) of the first membrane (30), and measuring a second concentration of the analyte diffused through the second contact surface (31a) of the second membrane (31), and determining the concentration of said analyte in the blood or tissue using said first and second concentration.

**Fig. 1**

**Fig. 2**

Fig. 3A

Fig. 3B

Fig. 3C

Fig. 3D

**Fig. 3E**

**Fig. 3F**

EP 4 706 527 A1

Fig. 3G

Fig. 3H

Fig. 3I

19

**Fig. 4A**

**Fig. 4B**

**Fig. 5**

**Fig. 6**

| | Europäisches Patentamt |
| | European Patent Office |
| | Office européen des brevets |

**EUROPEAN SEARCH REPORT**

**Application Number**

EP 24 19 9102

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2016/008898 A1 (UNIV ZUERICH [CH] ET AL.) 21 January 2016 (2016-01-21)<br>* figures 1, 4 *<br>* page 21, line 12 - line 35 * | 1-15 | INV.<br>A61B5/145<br>A61B5/1468<br>A61B5/1495<br>A61B5/00 |
| A | US 2023/113175 A1 (GARAI ELLIS [US] ET AL) 13 April 2023 (2023-04-13)<br>* paragraph [0053] * | 1-15 | |
| A | US 2022/202327 A1 (LIU ZENGHE [US] ET AL) 30 June 2022 (2022-06-30)<br>* paragraph [00273] *<br>* figure 20 * | 1-15 | |
| A | SHENG LUO XIE ET AL: "TOWARDS AN IMPLANTABLE AND REFILLABLE GLUCOSE SENSOR BASED ON OXYGEN ELECTRODE PRINCIPLES", SENSORS AND ACTUATORS B: CHEMICAL, ELSEVIER BV, NL, vol. B17, no. 2, 1 January 1994 (1994-01-01), pages 133-142, XP000428656, ISSN: 0925-4005<br>* figures 1-2 * | 1-15 | |
| A | US 2021/156815 A1 (MUCIC ROBERT [US] ET AL) 27 May 2021 (2021-05-27)<br>* paragraph [0026] - paragraph [0029] * | 1-15 | TECHNICAL FIELDS SEARCHED (IPC)<br>A61B |
| A | US 2024/074682 A1 (FERNANDEZ DE AVILA BERTA ESTEBAN [US] ET AL) 7 March 2024 (2024-03-07)<br>* paragraph [0165] *<br>* figures 1A-I * | 1-15 | |

-/--

The present search report has been drawn up for all claims

| Place of search | Date of completion of the search | Examiner |
|---|---|---|
| The Hague | 15 January 2025 | Knoop, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

**EUROPEAN SEARCH REPORT**

Europäisches
Patentamt
European
Patent Office
Office européen
des brevets

Application Number

EP 24 19 9102

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2016/129894 A1 (KWANGWOON UNIV INDUSTRY-ACADEMIC COLLABORATION FOUND [KR] ET AL.) 18 August 2016 (2016-08-18) * figure 3 * ----- | 1-15 | |
| A | US 2018/292348 A1 (SALZER COREY [US] ET AL) 11 October 2018 (2018-10-11) * paragraph [0002] * ----- | 1-15 | |

TECHNICAL FIELDS
SEARCHED      (IPC)

The present search report has been drawn up for all claims

| Place of search | Date of completion of the search | Examiner |
|---|---|---|
| The Hague | 15 January 2025 | Knoop, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 19 9102

This annex lists the patent family members relating to the patent documents cited in the above-mentioned European search report.
The members are as contained in the European Patent Office EDP file on
The European Patent Office is in no way liable for these particulars which are merely given for the purpose of information.

15-01-2025

| Patent document cited in search report | | | Publication date | Patent family member(s) | | | Publication date |
|---|---|---|---|---|---|---|---|
| WO 2016008898 | A1 | | 21-01-2016 | CN | 106714686 | A | 24-05-2017 |
| | | | | EP | 2974656 | A1 | 20-01-2016 |
| | | | | EP | 3169235 | A1 | 24-05-2017 |
| | | | | JP | 6983065 | B2 | 17-12-2021 |
| | | | | JP | 2017526908 | A | 14-09-2017 |
| | | | | US | 2017202492 | A1 | 20-07-2017 |
| | | | | WO | 2016008898 | A1 | 21-01-2016 |
| US 2023113175 | A1 | | 13-04-2023 | CN | 115944294 | A | 11-04-2023 |
| | | | | EP | 4162874 | A1 | 12-04-2023 |
| | | | | US | 2023113175 | A1 | 13-04-2023 |
| US 2022202327 | A1 | | 30-06-2022 | AU | 2022205038 | A1 | 22-06-2023 |
| | | | | CA | 3202248 | A1 | 07-07-2022 |
| | | | | EP | 4271826 | A1 | 08-11-2023 |
| | | | | JP | 2024501695 | A | 15-01-2024 |
| | | | | US | 2022202327 | A1 | 30-06-2022 |
| | | | | WO | 2022147506 | A1 | 07-07-2022 |
| US 2021156815 | A1 | | 27-05-2021 | NONE | | | |
| US 2024074682 | A1 | | 07-03-2024 | US | 2024074682 | A1 | 07-03-2024 |
| | | | | WO | 2024050124 | A1 | 07-03-2024 |
| WO 2016129894 | A1 | | 18-08-2016 | KR | 20160097639 | A | 18-08-2016 |
| | | | | WO | 2016129894 | A1 | 18-08-2016 |
| US 2018292348 | A1 | | 11-10-2018 | CN | 108139351 | A | 08-06-2018 |
| | | | | EP | 3359959 | A1 | 15-08-2018 |
| | | | | US | 2018292348 | A1 | 11-10-2018 |
| | | | | US | 2021102914 | A1 | 08-04-2021 |
| | | | | WO | 2017062849 | A1 | 13-04-2017 |

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016008898 A

- WO 2016008898 A1

**Non-patent literature cited in the description**

- **COURTEN, D.** ; **BAUMANN, L.** ; **SCHERER, L. J.** ; **WOLF, M.** Opto-Fluidic Chip for Continuous Inline Monitoring of Glucose with Kinetic Enzymatic Fluorescence Detection. *Procedia Engineering*, 2012, vol. 47, 1203-1206